# EUROPEAN PATENT SPECIFICATION

(11) **EP 4 119 044 B1**
(45) Date of publication and mention of the grant of the patent: **10.12.2025**
(21) Application number: 20923977.1
(22) Date of filing: 13.03.2020
(51) Int. Cl.: A61B 5/05, A61B 5/055, A61B 5/243, A61B 5/00, A61B 5/06, G16H 30/20, G16H 40/63, G16H 50/20, G06T 5/50, A61B 34/20, A61B 90/00, A61B 18/12, A61N 1/06, A61B 6/00, G16H 30/40

(54) **MEDICAL DEVICE**
MEDIZINISCHE VORRICHTUNG
DISPOSITIF MÉDICAL

(43) Date of publication of application: 18.01.2023
(73) Proprietor: Asahi Intecc Co., Ltd., Seto-shi, Aichi 489-0071 (JP)
(72) Inventor: OSHIMA, Fumiyoshi, Seto-shi, Aichi 489-0071 (JP)
(74) Representative: Winter, Brandl - Partnerschaft mbB
(86) International application number: PCT/JP2020/011024
(87) International publication number: WO 2021/181648

(56) References cited:
- WO-A1-2005/117695
- DE-A1- 4 215 901
- JP-A- 2017 113 559
- JP-A- H10 230 016
- US-A1- 2003 018 277
- US-A1- 2004 077 964
- US-A1- 2016 367 161
- ACHENBACH S ET AL: "Magnetocardiography: clinical investigations with a biomagnetic multichannel system", PHYSIOLOGICAL MEASUREMENT, INSTITUTE OF PHYSICS PUBLISHING, BRISTOL, GB, vol. 14, no. 4A, 1 November 1993 (1993-11-01), pages A61 - A68, XP020073950, ISSN: 0967-3334, DOI: 10.1088/0967-3334/14/4A/011

## Description

### TECHNICAL FIELD

The disclosed embodiments relate to a medical apparatus and an image generation method.

### BACKGROUND ART

There are conventionally known techniques of visually representing the state of an organ in a living body. For example, JP 4597329 discloses the technique of estimating a current vector in the region cell surrounded by three points on the heart from a measurement result obtained from the three points, displaying the obtained vector group on the image of the heart surface, and displaying the measurement result as a pseudo color map.

Further relevant prior art is provided by the following publications. DE 42 15 901 A1 discloses a catheter with an embedded magnetic field generator for determining its position in the body using magnetic sensors. In ACHENBACH S ET AL: "Magnetocardiography: clinical investigations with a biomagnetic multichannel system",PHYSIOLOGICAL MEASUREMENT, INSTITUTE OF PHYSICS PUBLISHING, BRISTOL, GB, vol. 14, no. 4A, 1 November 1993, pages A61-A68, ISSN: 0967-3334, DOI: 10.1088/0967-3334/14/4A/011, a technique is disclosed to perform non-invasive localisation of cardiac lesions using magnetocardiographic signals fused with MR images. US 2016/0367161 A1 discloses automatic image fusion of anatomical and electrophysiological data to assist in mapping and guiding cardiac interventions. US 2004/0077964 A1 describes a system for identifying intramyocardial electrical reentry circuits by superimposing current density maps from magnetocardiographic data onto anatomical images. US 2003/0018277 A1 discloses integration of magnetic tracking of a pacemaker lead with anatomical imaging and electrogram data to guide implantation.

### SUMMARY OF INVENTION

### TECHNICAL PROBLEM

For example, for treatment of arrhythmia, etc., there is a demand to improve the technique of providing treatment while visually checking the state of the organ including a lesion (arrhythmia site). However, even with the above-described conventional art, there is still room for improvements in the technique of displaying the state of the organ including the lesion.

The disclosed embodiments have been made to solve the above-described problem and have an object to provide the technique to improve the technique of displaying the state of the organ including the lesion.

### SOLUTION TO PROBLEM

The invention is defined in independent claim 1. The disclosed embodiments have been made to solve at least part of the above-described problem and may be implemented as the aspects below.
(1) According to one aspect of the disclosed embodiments, a medical apparatus is provided. The medical apparatus includes a biomagnetic field information acquisition portion that acquires biomagnetic field information obtained from a biomagnetic field generated by an organ including a lesion in a living body, a lesion position information detection portion that detects position information on the lesion in the organ from the acquired biomagnetic field information, an image information acquisition portion that acquires image information including a magnetic resonance imaging (MRI) image or computerized tomography (CT) image of the organ, and a synthetic image generation portion that uses the image information and the position information on the lesion to generate a synthetic image including a three-dimensional or two-dimensional organ model image of the organ and an image representing a position of the lesion.
   With this configuration, the medical apparatus displays the synthetic image including the three-dimensional or two-dimensional organ model image of the organ and the image representing the position of the lesion, and therefore the technician may specify the position of the lesion visually and easily. Thus, this configuration may improve the technique of displaying the state of the organ including the lesion.
(2) In the medical apparatus according to the above aspect, the biomagnetic field information may include information about a magnetic field strength distribution of the biomagnetic field generated by the organ, and the synthetic image generation portion may use the biomagnetic field information, the image information, and the position information on the lesion to generate a synthetic image including a three-dimensional or two-dimensional magnetic field strength distribution image of the organ, the three-dimensional or two-dimensional organ model image of the organ, and the image representing the position of the lesion. With this configuration, the medical apparatus displays the synthetic image further including the three-dimensional or two-dimensional magnetic field strength distribution image of the organ as well as the three-dimensional or two-dimensional organ model image of the organ and the image representing the position of the lesion. This makes it possible for the technician to understand the state of the organ in further detail visually and easily. Thus, this configuration may further improve the technique of displaying the state of the organ including the lesion.
(3) The medical apparatus according to the above aspect may further include a device magnetic field information acquisition portion that acquires device magnetic field information obtained from a magnetic field generated by a medical device inserted into the living body, and a device position information detection portion that detects position information on the medical device in the living body from the acquired device magnetic field information, wherein the synthetic image generation portion may use the position information on the medical device, the image information, and the position information on the lesion to generate a synthetic image including an image representing a position of the medical device, the three-dimensional or two-dimensional organ model image of the organ, and the image representing the position of the lesion. With this configuration, the medical apparatus may display the synthetic image further including the image representing the position of the medical device as well as the three-dimensional or two-dimensional organ model image of the organ and the image representing the position of the lesion. This makes it possible for the technician to understand the relative position of the medical device with respect to the lesion visually and easily. Thus, this configuration may further improve the technique of displaying the state of the organ including the lesion.
(4) In the medical apparatus according to the above aspect, the biomagnetic field information may be information output from a magnetic sensor that detects the biomagnetic field generated by the organ. With this configuration, the output from the magnetic sensor may be used to easily detect the position of the lesion in the organ.
(5) In the medical apparatus according to the above aspect, the device magnetic field information may be information output from a magnetic sensor that detects a magnetic field generated by a magnetic body provided in a distal end of the medical device, and the position of the medical device may be a position of the magnetic body of the medical device. With this configuration, the output from the magnetic sensor may be used to easily detect the position of the magnetic body provided in the distal end of the medical device.
(6) The medical apparatus according to the above aspect may further include a display portion that displays the synthetic image generated by the synthetic image generation portion. With this configuration, the technician may easily specify the position of the lesion by checking the synthetic image displayed on the display portion.
(7) According to another aspect of the disclosed embodiments, a medical apparatus is provided. The medical apparatus includes a device magnetic field information acquisition portion that acquires device magnetic field information obtained from a magnetic field generated by a medical device inserted into a living body, a device position information detection portion that detects position information on the medical device in the living body from the acquired device magnetic field information, an image information acquisition portion that acquires image information including an MRI image or CT image of an organ in the living body, and a synthetic image generation portion that uses the position information on the medical device and the image information to generate a synthetic image including an image representing a position of the medical device and a three-dimensional or two-dimensional organ model image of the organ. With this configuration, the medical apparatus displays the synthetic image including the three-dimensional or two-dimensional organ model image of the organ and the image representing the position of the medical device, and therefore the technician may specify the position of the medical device visually and easily.

Furthermore, the disclosed embodiments may be realized in various modes and may be realized in modes such as image generation devices, image generation methods, examination apparatuses, examination methods, methods for manufacturing medical apparatuses, computer programs, etc.

### BRIEF DESCRIPTION OF DRAWINGS

Fig. 1 is an explanatory diagram illustrating a schematic configuration of a medical apparatus according to a first embodiment.
Fig. 2 is a functional block diagram of a main control portion and a synthetic image generation portion.
Fig. 3 is a diagram illustrating a three-dimensional heart model and an organ model image.
Fig. 4 is a diagram schematically illustrating a method for detecting a biomagnetic field by a magnetic sensor array.
Fig. 5 is a diagram illustrating a magnetic field strength distribution image.
Fig. 6 is an explanatory diagram illustrating the magnetic field strength distribution image on a plurality of virtual planes of a heart.
Fig. 7 is an explanatory diagram illustrating a three-dimensional magnetic field strength distribution model of the heart.
Fig. 8 is a diagram illustrating a synthetic image of the organ model image and the magnetic field strength distribution image.
Fig. 9 is a diagram illustrating a relationship between a flow path of an electric signal in a healthy heart and an electrocardiogram.
Fig. 10 is a diagram illustrating a lesion position image.
Fig. 11 is an explanatory diagram schematically illustrating a method for detecting a living-body/device mixed magnetic field.
Fig. 12 is a diagram illustrating a device position image.
Fig. 13 is an explanatory diagram illustrating the device position images on the plurality of virtual planes of the heart.
Fig. 14 is a schematic diagram illustrating a method for generating the synthetic image.
Fig. 15 is an explanatory diagram illustrating a state of displaying the organ model image on a display screen.
Fig. 16 is an explanatory diagram illustrating the synthetic image of the organ model image and another image.
Fig. 17 is an explanatory diagram illustrating the synthetic image of the organ model image and other two images.
Fig. 18 is an explanatory diagram illustrating the synthetic image of the organ model image and other three images.

### EMBODIMENTS OF THE INVENTION

### <First Embodiment>

### <1. Overall Configuration of Medical Apparatus 1>

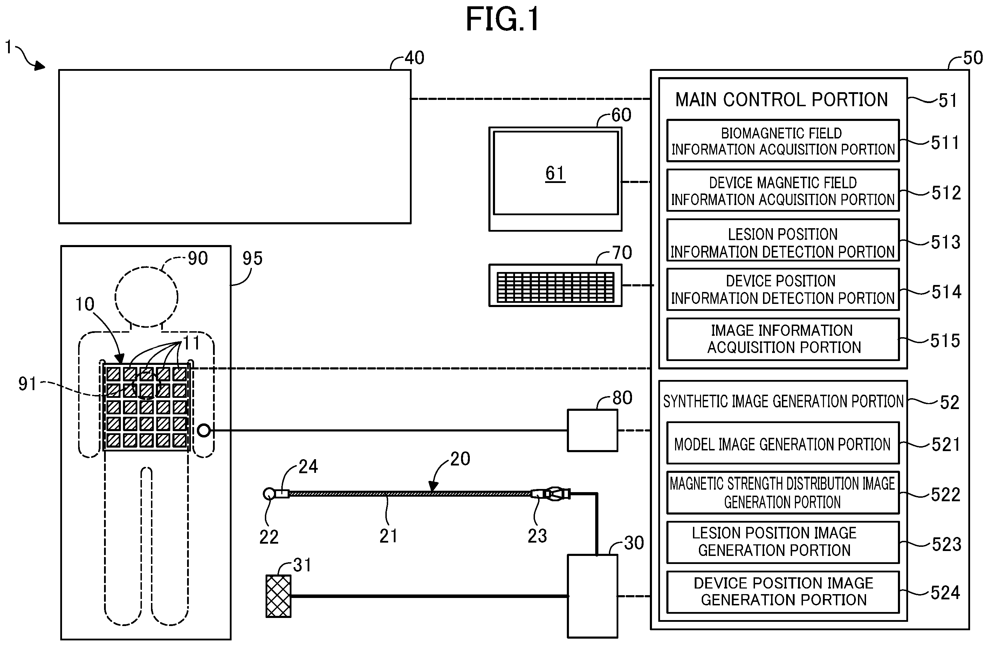

Fig. 1 is an explanatory diagram illustrating a schematic configuration of a medical apparatus 1 according to a first embodiment. The medical apparatus 1 is an apparatus used to provide treatment for a living body (here, a human body) 90, which is the target for treatment, and includes a magnetic sensor array 10, a catheter 20, a high-frequency generator 30, a CT device 40, a computer 50, a monitor 60, an operating portion 70, and an electrocardiograph 80. Here, the medical apparatus 1 used for arrhythmia treatment is described as an example according to the present embodiment.

The magnetic sensor array 10 is a device that detects the strength, orientation, and the like, of the biomagnetic field generated by the human body 90, which is the target for treatment, and is arranged in a matrix such that a plurality of magnetic sensors 11 is arranged vertically and horizontally. The magnetic sensor 11 is a device that detects the strength and orientation of the biomagnetic field, and examples thereof may include a GSR (GHz-Spin-Rotation) sensor, a magnetoresistive effect device (MR), a magnetic impedance device (MI), and a superconducting quantum interference device (SUQUID). Here, the magnetic sensor array 10 is located near a center portion of a table (bed) 95 where the human body 90 lies during treatment. Furthermore, the magnetic sensor array 10 may be configured to be attached to the human body 90 during treatment. For example, the magnetic sensor array 10 may be configured to have a band shape to be wrapped around the human body 90 or may be configured to have a garment or cap shape. In these cases, the magnetic sensor 11 may be provided along the shape of the human body 90. Further, the plate-shaped magnetic sensor arrays 10 may be provided in three dimensions on one or both of the front surface and the back surface and one or both of the side surfaces of the human body, respectively. Here, an example of detecting the strength and orientation of the heart magnetic field generated by a heart 91, which is one of the organs of the human body 90, is described.

The catheter 20 is what is called an cautery catheter that is inserted into the human body 90 during treatment and generates plasma from a distal end inside the heart 91. The catheter 20 includes a main body portion 21, a distal tip 22, a connector 23, and a marker 24. The main body portion 21 has an elongated outer shape and has a first wire (core wire) and a second wire, not illustrated, each having conductivity and provided inside an electrical insulating outer layer. The distal tip 22 is provided in the distal end of the main body portion 21 and is electrically connected to a distal end of the first wire. The connector 23 is provided in a proximal end of the main body portion 21 and is coupled to the high-frequency generator 30. The coupling between the connector 23 and the high-frequency generator 30 causes the respective proximal ends of the first wire and the second wire to be electrically connected to the high-frequency generator 30. The marker 24 is a conductive magnetic member used to detect the position of a distal end portion of the catheter 20 and is provided on the distal end side of the main body portion 21 and at the proximal end side of the distal tip 22. Here, the marker 24 is electrically connected to a distal end of the second wire.

The high-frequency generator 30 is a device that supplies a high-frequency current to the catheter 20 and that supplies a high-frequency current to the distal tip 22 via the first wire and supplies a position-detection current to the marker 24 via the second wire. The high-frequency generator 30 is also electrically connected to a conducting return electrode 31 to supply a high-frequency current to the distal tip 22 and thus generate plasma between the distal tip 22 and the conducting return electrode 31. This plasma may cauterize the portion (lesion) of the heart 91 having arrhythmia. The high-frequency generator 30 supplies the position-detection current to the marker 24 to generate a magnetic field from the marker 24. This makes it possible to specify the position and orientation of the distal end portion of the catheter 20, as described below. Here, the high-frequency generator 30 is coupled to the computer 50 to switch on/off supply of the high-frequency current to the distal tip 22 and supply of the position-detection current to the marker 24 in accordance with an instruction from the computer 50.

The CT device 40 includes, inside a gantry (mount), a tube that emits X-rays and an arc-shaped detector that detects X-rays to generate a CT image representing the shape of the heart 91 when the tube rotates by 360° around the human body 90 lying on the bed 95 and output image information including the CT image to the computer 50. Furthermore, the medical apparatus 1 may include an MRI device instead of the CT device as the device that generates images representing the shape of an organ inside the human body 90. That is, the medical apparatus 1 may acquire the image information including an MRI image instead of the image information including the CT image.

The computer 50 is a device that controls the overall medical apparatus 1 and is electrically connected to the magnetic sensor array 10, the high-frequency generator 30, the CT device 40, the monitor 60, the operating portion 70, and the electrocardiograph 80. The computer 50 includes a CPU, a ROM, and a RAM, not illustrated, and performs the functions of a main control portion 51 and a synthetic image generation portion 52 when the CPU executes a program stored in the ROM.

The main control portion 51 exchanges information with the magnetic sensor array 10, the high-frequency generator 30, the CT device 40, the monitor 60, the operating portion 70, and the electrocardiograph 80 to control the overall medical apparatus 1. The main control portion 51 includes a biomagnetic field information acquisition portion 511, a device magnetic field information acquisition portion 512, a lesion position information detection portion 513, a device position information detection portion 514, and an image information acquisition portion 515. The functions of these functional portions will be described below.

When receiving a predetermined operation via the operating portion 70, the main control portion 51 controls the high-frequency generator 30 to supply the high-frequency current to the distal tip 22. Furthermore, when the high-frequency current is not supplied to the distal tip 22, the main control portion 51 intermittently supplies the position detection current to the marker 24. The main control portion 51 acquires, from the magnetic sensor array 10, information (hereinafter also referred to as "first magnetic field information") about the strength and orientation of the magnetic field detected by the magnetic sensor array 10 when the current is supplied to neither the distal tip 22 nor the marker 24 and information (hereinafter also referred to as "second magnetic field information") about the strength and orientation of the magnetic field detected by the magnetic sensor array 10 when the detection current is supplied to the marker 24.

The first magnetic field information is the biomagnetic field information representing the strength and orientation of a biomagnetic field MFh generated by the human body 90. The biomagnetic field MFh includes the magnetic field generated by an organ (e.g., the heart 91) inside the human body 90 and, when there is a lesion in the organ, the biomagnetic field MFh changes due to the lesion. That is, the first magnetic field information includes information about the lesion in the organ, and the first magnetic field information may be used to specify the position of the lesion in the organ. Therefore, it can be said that the first magnetic field information includes the position information on the lesion.

The second magnetic field information represents the strength and orientation of a magnetic field (hereinafter also referred to as "living-body/device mixed magnetic field") combining both the biomagnetic field MFh generated by the human body 90 and a magnetic field (hereinafter also referred to as "device magnetic field") MFm generated by the marker 24 of the catheter 20. As the second magnetic field information includes the information (hereinafter also referred to as "device magnetic field information") about the device magnetic field, the second magnetic field information may be used to specify the position of the catheter 20 (the marker 24) inside the human body 90. Thus, it can be said that the second magnetic field information includes the position information on the catheter 20.

The main control portion 51 controls the CT device 40 to acquire the information (hereinafter also referred to as "image information") including the CT image of the human body 90. That is, the main control portion 51 functions as what is called a console of the CT device 40. The main control portion 51 controls the electrocardiograph 80 to acquire, from the electrocardiograph 80, the information (hereinafter also referred to as "electrocardiographic information") including the electrocardiogram of the human body 90.

The synthetic image generation portion 52 uses the first magnetic field information (the biomagnetic field information) and the second magnetic field information (the position information on the catheter 20) output from the magnetic sensor array 10 and the image information output from the CT device 40 to generate a synthetic image CI described below. The synthetic image generation portion 52 displays the generated synthetic image CI on a display screen 61 of the monitor 60. The synthetic image generation portion 52 includes a model image generation portion 521, a magnetic field strength distribution image generation portion 522, a lesion position image generation portion 523, and a device position image generation portion 524. The details of each functional portion will be described below.

The monitor 60 is a display portion including the display screen 61 and includes a liquid crystal display, or the like. The medical apparatus 1 may include a display portion other than the monitor 60. For example, the medical apparatus 1 may include smart glasses including a display screen or a projector that projects images. The operating portion 70 includes a keyboard, or the like, and is operated, for example, when a technician of the catheter 20 switches the display content of the display screen 61. The operating portion 70 may be provided on part of the catheter 20.

The electrocardiograph 80 includes four limb leads attached to the four limbs of the human body 90 and six chest leads attached to the chest and outputs the information (electrocardiographic information) including the electrocardiogram (here, 12-lead electrocardiogram) of the human body 90 to the computer 50.

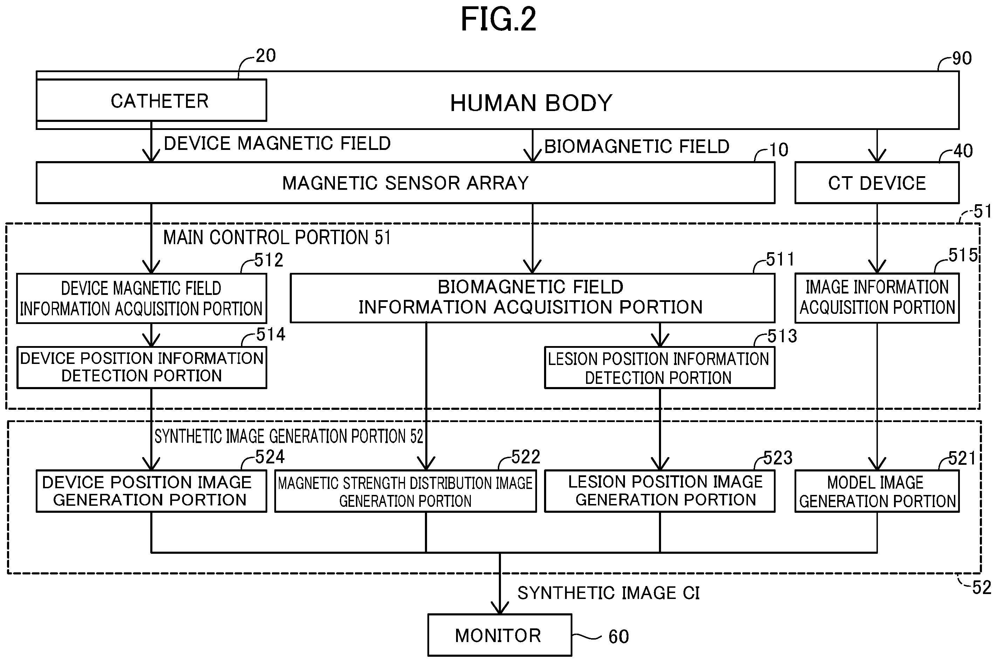

Fig. 2 is a functional block diagram of the main control portion 51 and the synthetic image generation portion 52. The biomagnetic field information acquisition portion 511 of the main control portion 51 controls the magnetic sensor array 10 to acquire the first magnetic field information from the magnetic sensor array 10. Here, the magnetic sensor array 10 detects the biomagnetic field (heart magnetic field) generated by the heart 91 (Fig. 1), and the first magnetic field information includes the information about the biomagnetic field generated by the heart 91. The acquired first magnetic field information is stored in a storage portion, not illustrated, of the computer 50. The lesion position information detection portion 513 detects the position information on the lesion (the portion having arrhythmia) of the heart 91 from the acquired first magnetic field information.

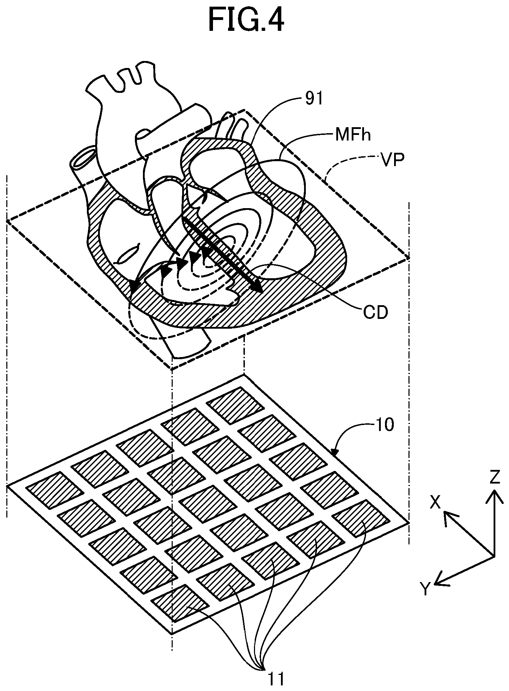

The magnetic field strength distribution image generation portion 522 of the synthetic image generation portion 52 generates a three-dimensional or two-dimensional magnetic field strength distribution image (also referred to as "magnetocardiogram image" or "biomagnetic field distribution image") MI of the heart 91 from the first magnetic field information acquired by the biomagnetic field information acquisition portion 511. An example of the method for generating the magnetic field strength distribution image MI from the first magnetic field information will be described below using Fig. 4 to Fig. 7.

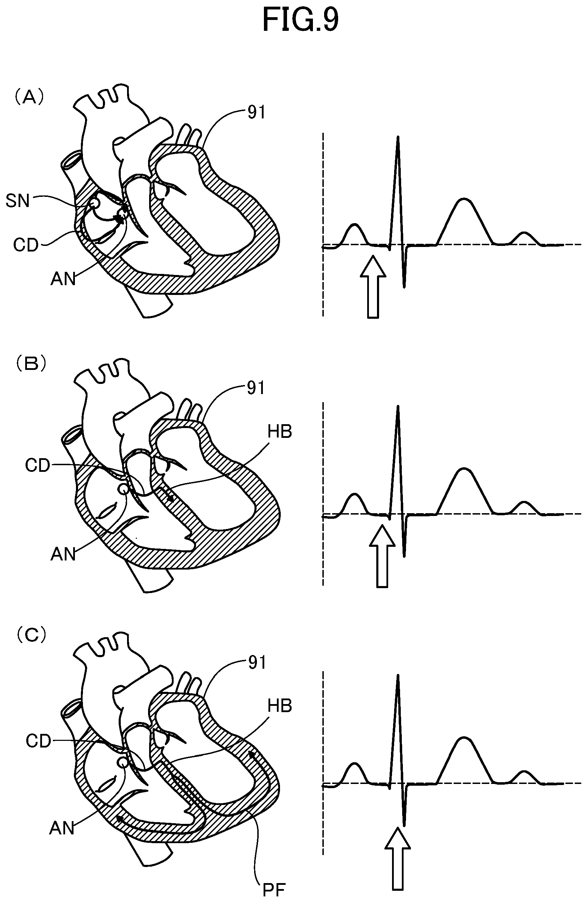

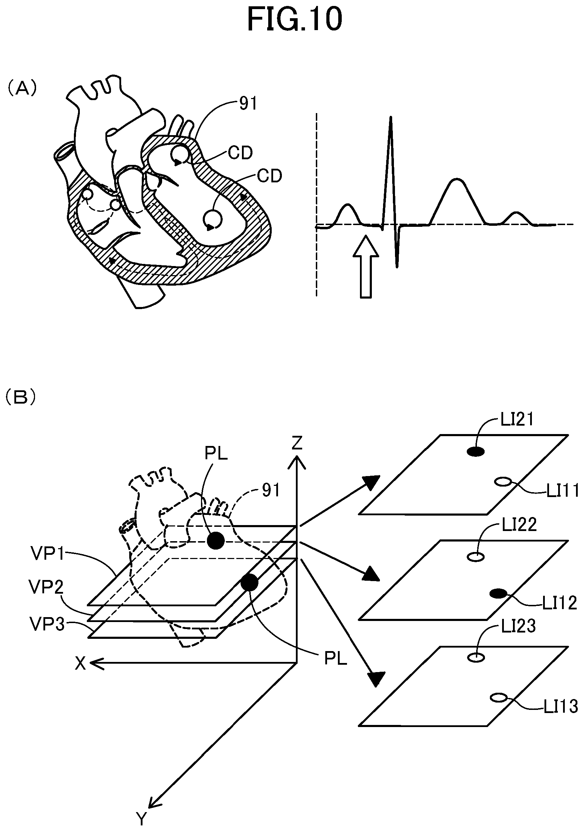

The lesion position image generation portion 523 of the synthetic image generation portion 52 uses the position information on the lesion detected by the lesion position information detection portion 513 to generate a lesion position image LI representing the position of the lesion. An example of the method for generating the lesion position image LI by detecting the position information on the lesion from the first magnetic field information will be described below using Fig. 9 and Fig. 10.

The device magnetic field information acquisition portion 512 of the main control portion 51 controls the magnetic sensor array 10 to acquire the second magnetic field information from the magnetic sensor array 10. As the second magnetic field information includes the biomagnetic field information and the information (device magnetic field information) about the device magnetic field, the device magnetic field information acquisition portion 512 acquires the device magnetic field information from the second magnetic field information. The acquired second magnetic field information is stored in the storage portion of the computer 50. The device position information detection portion 514 detects, from the acquired device magnetic field information, the information (device position information) representing the position of the catheter 20 inside the human body 90.

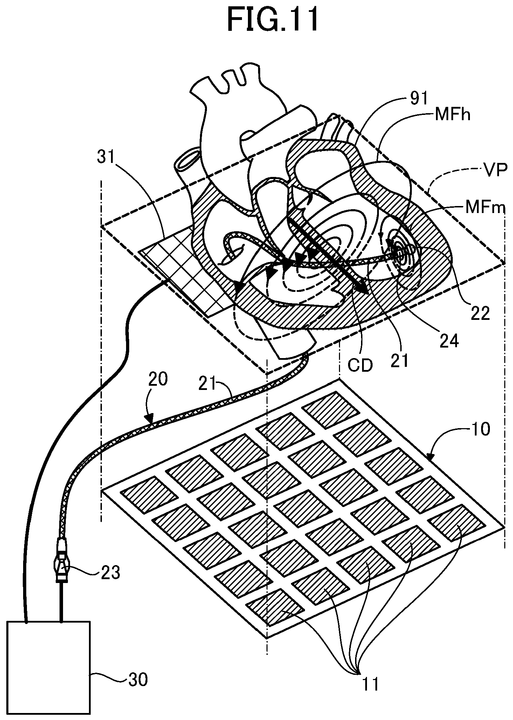

The device position image generation portion 524 of the synthetic image generation portion 52 uses the device position information detected by the device position information detection portion 514 to generate a device position image PI representing the position of the catheter 20 (the marker 24). An example of the method for generating the device position image PI by detecting the device position information from the second magnetic field information including the device magnetic field information will be described below using Fig. 11 to Fig. 13.

The image information acquisition portion 515 of the main control portion 51 controls the CT device 40 to acquire the image information including the CT image from the CT device 40. The acquired image information is stored in the storage portion of the computer 50. Here, the information including the CT image of the heart 91 is acquired. Specifically, the image information acquisition portion 515 captures the entire heart 91 in cross-section at a time interval and acquires the image information including the cross-sections of the entire heart 91 at the time interval. Furthermore, in addition to the method for acquiring the image information directly by controlling the CT device 40, the image information acquisition portion 515 may also acquire the image information via a storage medium that stores the previously acquired image information.

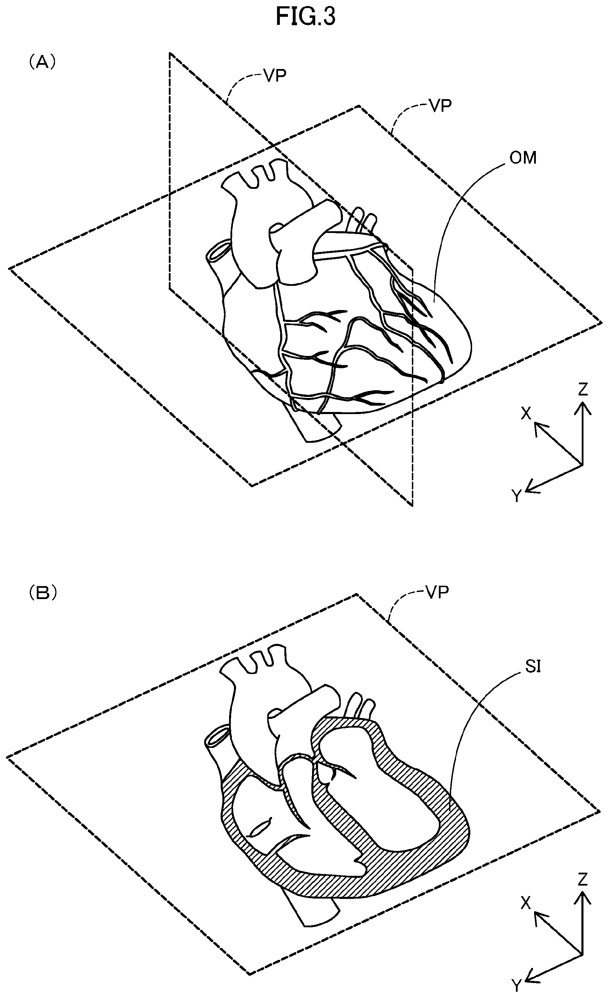

The model image generation portion 521 of the synthetic image generation portion 52 generates a three-dimensional or two-dimensional organ model image SI from the image information acquired by the image information acquisition portion 515. An example of the method for generating the organ model image SI from the image information will be described below using Fig. 3.

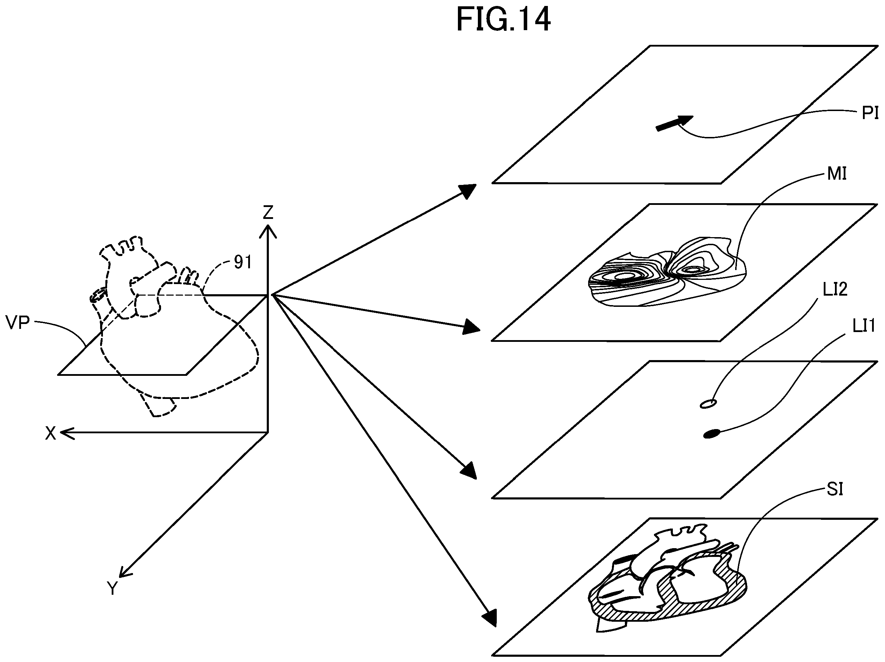

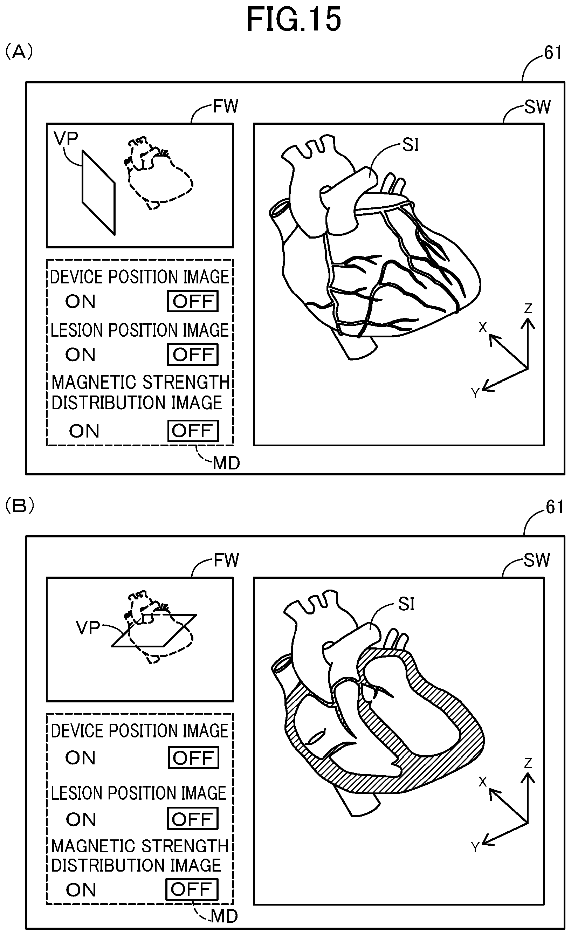

The synthetic image generation portion 52 superimposes any images among the organ model image SI, the magnetic field strength distribution image MI, the lesion position image LI, and the device position image PI to generate the synthetic image CI. The contents of the organ model image SI, the magnetic field strength distribution image MI, the lesion position image LI, and the synthetic image CI will be described below using Fig. 14. The generated synthetic image CI is displayed on the display screen 61 of the monitor 60. The synthetic image CI displayed on the display screen 61 will be described below using Fig. 15 to Fig. 18.

### <2. Organ Model Image SI>

Fig. 3 is a diagram illustrating a three-dimensional heart model OM and the organ model image SI. This describes the method for generating the two-dimensional organ model image (heart model image) SI from the image information including the CT image representing the shape of the heart 91 by the model image generation portion 521 (Fig. 2). The model image generation portion 521 first generates the three-dimensional organ model (three-dimensional heart model) OM illustrated in Fig. 3(A) from the image information acquired by the image information acquisition portion 515. The three-dimensional organ model OM is stereoscopic image data representing the external and internal shapes of the heart 91. An existing technique is applicable as the technique of generating the three-dimensional organ model OM from the image information including the CT image.

The model image generation portion 521 integrates cross-sectional images (a plurality of successive CT images) of the entire heart 91 acquired by the image information acquisition portion 515 at certain times to generate the three-dimensional organ model (three-dimensional heart model) OM of the heart 91 at the certain times. The synthetic image generation portion 52 integrates the cross-sectional images of the entire heart 91 at the respective times to generate the dynamic three-dimensional heart model OM that deforms over time. The synthetic image generation portion 52 uses the three-dimensional heart model OM to generate, as the organ model image (heart model image) SI, the three-dimensional heart model OM viewed from a virtual plane VP set at any position. The position and orientation of the virtual plane VP are set by an operation of the operating portion 70. For example, when the set virtual plane VP intersects with the three-dimensional heart model OM, the organ model image SI representing the cross-section of the three-dimensional heart model OM is generated as illustrated in Fig. 3(B). When the set virtual plane VP does not intersect with the three-dimensional heart model OM, the organ model image SI representing the appearance (outer surface) of the three-dimensional heart model OM viewed from the virtual plane VP is generated. The synthetic image generation portion 52 may generate both the two-dimensional organ model image SI and the three-dimensional organ model image SI and, in accordance with the operation of the operating portion 70, generates the organ model image SI in the designated dimension. The two-dimensional organ model image SI is an image representing only the surface of the portion intersecting with the virtual plane VP in the three-dimensional heart model OM. The three-dimensional organ model image SI is an image also representing the portion of the three-dimensional heart model OM in the depth direction viewed from the virtual plane VP as well as the portion intersecting with the virtual plane VP.

The three-dimensional heart model OM includes the information related to the coordinate position of the portion corresponding to a specific site of the heart. Here, it includes information such as the position of the sinus node, the position of the atrioventricular node, the orientation of the His bundle, the position of a Purkinje fiber, etc. The coordinate positions and orientations of the portions corresponding to these specific sites may be specified by, for example, fitting with the contour image representing the typical positional relationship of these specific sites.

### <3. Magnetic field strength Distribution Image MI>

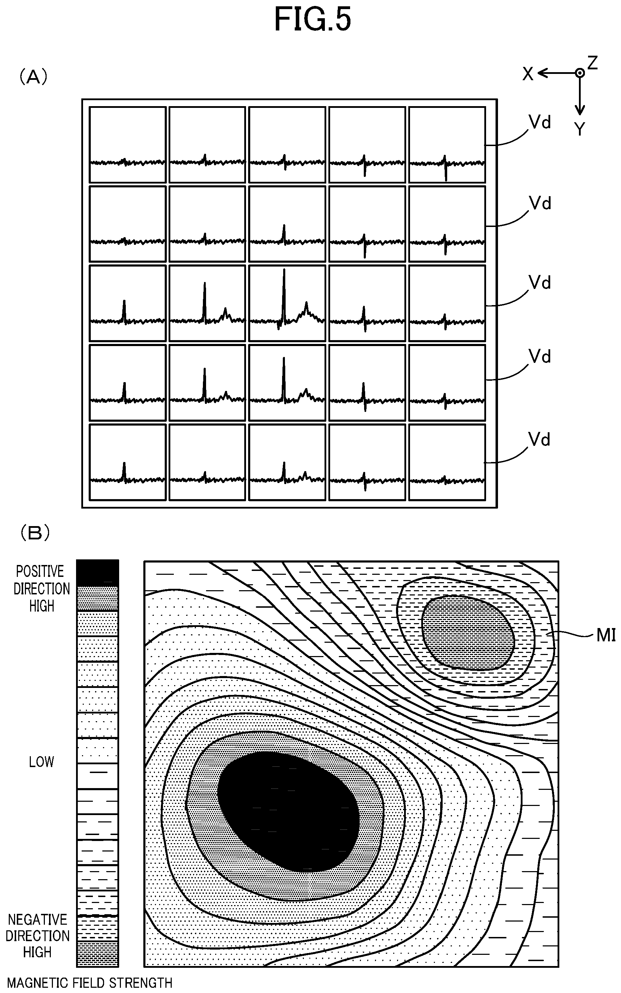

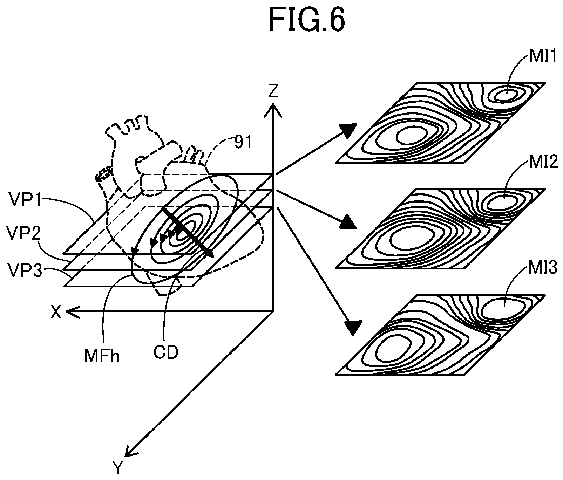

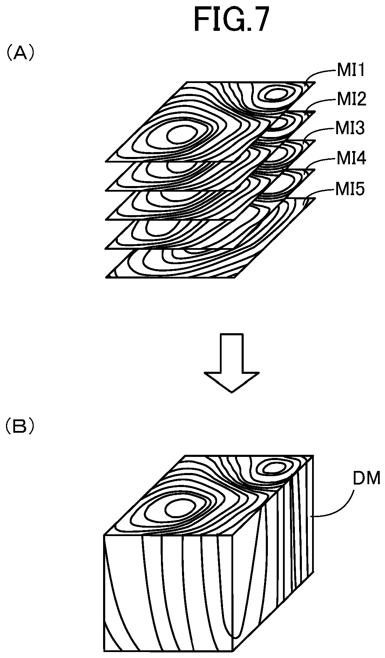

By using Fig. 4 to Fig. 7, the method for generating the magnetic field strength distribution image (magnetocardiogram image) MI by the magnetic field strength distribution image generation portion 522 will be described. Fig. 4 is an explanatory diagram schematically illustrating the method for detecting the biomagnetic field MFh by the magnetic sensor array 10. Fig. 5(A) is an explanatory diagram illustrating the strength (detection value Vd) of the biomagnetic field MFh detected by each of the magnetic sensors 11 of the magnetic sensor array 10. Fig. 5(B) is an explanatory diagram illustrating the magnetic field strength distribution image MI. Fig. 6 is an explanatory diagram illustrating the magnetic field strength distribution image MI on the plurality of virtual planes VP of the heart 91. Fig. 7 is an explanatory diagram illustrating a three-dimensional magnetic field strength distribution model DM of the heart 91. Here, it is assumed that the main control portion 51 does not supply the currents to both the distal tip 22 and the marker 24 and no magnetic field occurs from the distal tip 22 or the marker 24. Therefore, the magnetic sensor array 10 outputs the biomagnetic field information (the first magnetic field information) representing the strength and orientation of the biomagnetic field MFh generated by the human body 90.

As illustrated in Fig. 4, in the heart 91, the sinus node generates an electric signal CD to contract the atria and ventricles. The magnetic sensor array 10 detects the strength and orientation of the biomagnetic field (heart magnetic field) MFh generated due to the electric signal CD. In the magnetic sensor array 10, the magnetic sensors 11 are arranged in a matrix on a two-dimensional plane (XY plane), and therefore the strength (the detection value Vd) of the biomagnetic field MFh at each position on the two-dimensional plane may be detected as illustrated in Fig. 5(A). Fig. 5(A) illustrates time-series changes in the strength of the biomagnetic field MFh at each position on the two-dimensional plane (XY plane). From the temporal changes in the strength of the biomagnetic field MFh at each position on the two-dimensional plane, the orientation of the biomagnetic field MFh on the two-dimensional plane may be detected. Furthermore, the magnetic sensor 11 is configured to detect changes in the strength of the biomagnetic field MFh in the normal direction (Z-direction) of the two-dimensional plane. Here, each of the magnetic sensors 11 includes a plurality of (e.g., two) elements arranged in the normal direction of the two-dimensional plane so as to detect the strength of the biomagnetic field MFh at a position relatively close to the heart 91 in the normal direction (Z-direction) and the strength of the biomagnetic field MFh at a relatively far position. With this configuration, the magnetic sensor array 10 may detect the strength and orientation of the biomagnetic field MFh on the arbitrary virtual plane (XY plane) VP intersecting with the heart 91. The magnetic sensor array 10 outputs, to the biomagnetic field information acquisition portion 511, the biomagnetic field information (the first magnetic field information) including the strength of the biomagnetic fields MFh detected by the respective magnetic sensors 11.

The magnetic field strength distribution image generation portion 522 generates the magnetic field strength distribution image MI illustrated in Fig. 5(B) from the biomagnetic field information (the first magnetic field information) output from the magnetic sensor array 10. Here, as an example of the magnetic field strength distribution image MI, the strength of the biomagnetic field MFh at each position on the two-dimensional plane (XY plane) is represented in a contour. Furthermore, the strength of the biomagnetic field MFh may be represented by a method such as color gradation other than contours. As illustrated in Fig. 6, the magnetic field strength distribution image generation portion 522 may generate, from the biomagnetic field information (the first magnetic field information), the magnetic field strength distribution image MI on the arbitrary virtual plane VP intersecting with the heart 91. Here, as an example, magnetic field strength distribution images MI1, MI2, and MI3 corresponding to three virtual planes (a first virtual plane VP1, a second virtual plane VP2, and a third virtual plane VP3), respectively, are illustrated.

Furthermore, the magnetic field strength distribution image generation portion 522 according to the present embodiment integrates the magnetic field strength distribution images MI (a plurality of successive magnetic field strength distribution images) in the respective cross-sections of the entire heart 91 at certain times as illustrated in Fig. 7(A) to generate the three-dimensional magnetic field strength distribution model DM of the heart 91 at the certain times as illustrated in Fig. 7(B). Furthermore, the magnetic field strength distribution images MI in the respective cross-sections of the entire heart 91 at the respective times are integrated to generate the dynamic three-dimensional magnetic field strength distribution model DM that changes over time. The magnetic field strength distribution image generation portion 522 may generate both the two-dimensional magnetic field strength distribution image MI and the three-dimensional magnetic field strength distribution image MI. The two-dimensional magnetic field strength distribution image MI is the image representing the magnetic field strength distribution of the portion intersecting with the virtual plane VP in the three-dimensional magnetic field strength distribution model DM. The three-dimensional magnetic field strength distribution image MI is the image representing the magnetic field strength distribution of the entire three-dimensional magnetic field strength distribution model DM viewed from the virtual plane VP or the portion included in any spatial region in the three-dimensional magnetic field strength distribution model DM. Therefore, the depth direction of the magnetic field strength distribution is also represented in the three-dimensional magnetic field strength distribution image MI.

The three-dimensional magnetic field strength distribution model DM includes the information related to the coordinate position of the portion corresponding to a specific site of the heart as well as the information about the orientation and strength of the magnetic field at each coordinate position in the three-dimensional space. Here, the information such as the position of the sinus node, the position of the atrioventricular node, the orientation of the His bundle, and the position of the Purkinje fiber is included as in the three-dimensional heart model OM. The coordinate positions, orientations, and the like, of the portions corresponding to these specific sites may be specified from the changes in the magnetic field generated due to the electric signal CD. For example, the sinus node is a portion serving as the origin of the electric signal CD and the atrioventricular node is a portion serving as the relay point of the electric signal CD, and therefore they may be specified from the generation position of the electric signal CD, the flow direction of the electric signal, and the like.

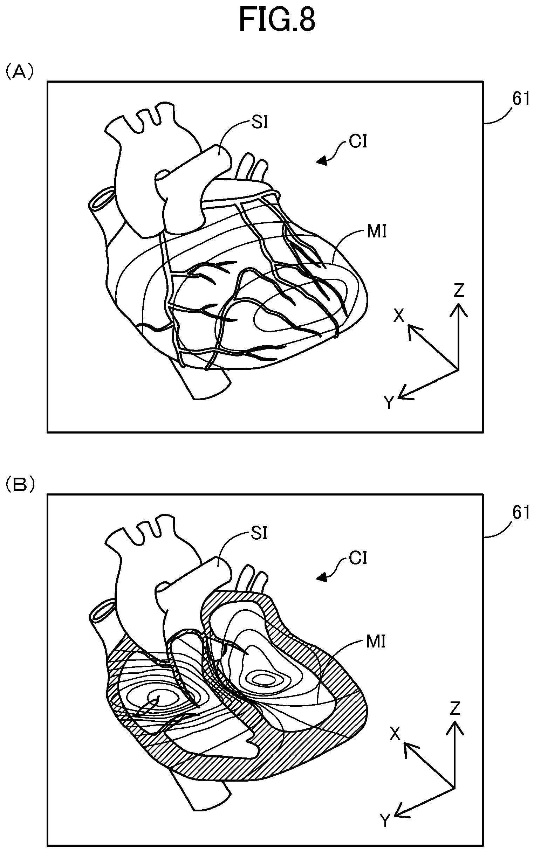

Fig. 8 is a diagram illustrating the synthetic image CI that is the synthesis of the organ model image SI and the magnetic field strength distribution image MI. The synthetic image generation portion 52 may generate the synthetic image CI that is the synthesis of the organ model image SI and the magnetic field strength distribution image MI. Specifically, as illustrated in Fig. 8(A), the synthetic image generation portion 52 may generate the synthetic image CI in which the magnetic field strength distribution image MI representing the magnetic field strength distribution of the outer surface portion of the heart 91 is superimposed on the three-dimensional organ model image SI representing the outer surface of the heart 91. Furthermore, as illustrated in Fig. 8(B), the synthetic image generation portion 52 may generate the synthetic image CI in which the magnetic field strength distribution image MI representing the magnetic field strength distribution of the cross-sectional portion of the heart 91 is superimposed on the three-dimensional organ model image SI representing the cross-section of the heart 91.

The alignment of the organ model image SI with the magnetic field strength distribution image MI may be executed by the alignment of the three-dimensional heart model OM with the three-dimensional magnetic field strength distribution model DM. As described above, the three-dimensional heart model OM and the three-dimensional magnetic field strength distribution model DM each include the information about the coordinate position of the specific site of the heart, and therefore fitting is applied to match each other's positions of the specific site so that the scales, positions, and inclinations of the organ model image SI and the magnetic field strength distribution image MI generated from these models may be matched.

### <4. Lesion Position Image LI>

By using Fig. 9 and Fig. 10, the method for generating the lesion position image LI by the lesion position image generation portion 523 will be described. Fig. 9 is an explanatory diagram illustrating the relationship between the flow path (nerve excitement propagation) of the electric signal CD in the heart 91 in a healthy state and the electrocardiogram. Fig. 10 is a diagram illustrating the lesion position image LI. The computer 50 previously stores the healthy subject biopotential magnetic information in which the biomagnetic field information for one cardiac cycle of the heart 91 in a healthy state is related to the electrocardiographic information (biopotential information). In the healthy subject biopotential magnetic information, for example, as illustrated in Figs. 9(A) to (C), a change in the biomagnetic field generated due to the electric signal CD flowing from a sinus node SN to an atrioventricular node AN, a His bundle HB, and a Purkinje fiber PF in that order is related to the position in the electrocardiogram at that time. The healthy subject biopotential magnetic information may be acquired from the heart 91 when the human body 90 is healthy or may be acquired from the heart of another healthy person.

The lesion position information detection portion 513 compares the first magnetic field information obtained from the magnetic sensor array 10 with the biomagnetic field information (changes in the biomagnetic field) included in the healthy subject biopotential magnetic information so as to specify the position of the lesion in the heart 91. The lesion position information detection portion 513 synchronizes the electrocardiographic information on the human body 90 obtained from the electrocardiograph 80 in real time with the electrocardiographic information included in the healthy person biopotential magnetic information so as to synchronize the first magnetic field information on the human body 90 obtained from the magnetic sensor array 10 in real time with the biomagnetic field information included in the healthy subject biopotential magnetic information. The lesion position information detection portion 513 uses the difference between the biomagnetic field at a certain time included in the first magnetic field information and the biomagnetic field at the identical timing included in the healthy subject biopotential magnetic information so as to specify the position of the lesion.

In the heart 91 including the lesion such as arrhythmia, the electric signal CD passes at a different timing through a different propagation route than normal as illustrated in Fig. 10(A) due to, for example, extrasystole that is the occurrence of an electric signal from a site other than the sinus node, bradycardia that is a slow heart rhythm when, for example, the propagation of the electric signal stops halfway, or tachycardia that is an earlier signal propagation than expected when, for example, there is an abnormal pathway of the electric signal. This results in a different (abnormal) biomagnetic field than normal. By obtaining the difference between the (normal) three-dimensional magnetic field strength distribution model included in the healthy subject biopotential magnetic information and the (abnormal) three-dimensional magnetic field strength distribution model DM generated from the first magnetic field information, it is possible to specify a position (three-dimensional position of the lesion) PL having arrhythmia in the heart 91 as illustrated in Fig. 10(B). The alignment of the three-dimensional magnetic field strength distribution model of the normal heart 91 at a certain time with the three-dimensional magnetic field strength distribution model of the abnormal heart 91 may be achieved by, for example, the alignment of the position of the specific site in each of the hearts (the position of the sinus node, the position of the atrioventricular node, the orientation of the His bundle, the position of the Purkinje fiber, etc.). Further, the method for specifying the three-dimensional position of the lesion may be a method other than the above-described method.

After the lesion position information detection portion 513 specifies the three-dimensional position PL of the lesion, the lesion position image generation portion 523 generates the lesion position image LI in Fig. 10(B). Here, the lesion position image LI is illustrated as an image having a circular shape. The lesion position image generation portion 523 may generate the lesion position image LI on the arbitrary virtual plane VP intersecting with the heart 91 from the first magnetic field information and the healthy subject biopotential magnetic information. Here, as an example, there are two lesions (the three-dimensional positions PL of the lesions) in the heart 91, and lesion position images LI11, LI12, LI13, and LI21, LI22, LI23 corresponding to three virtual planes (the first virtual plane VP1, the second virtual plane VP2, and the third virtual plane VP3), respectively, are illustrated. Here, the lesion position image generation portion 523 represents the position of the lesion in the Z-direction in the color of the lesion position image LI. Specifically, on the virtual plane (XY plane) VP that matches the position of the lesion in the Z-direction, the inner side of the circular shape of the lesion position image LI is illustrated in a first color (e.g., black), and on the virtual plane (XY plane) VP that does not match the position of the lesion in the Z-direction, the inner side of the circular shape of the lesion position image LI is illustrated in a second color (e.g., white). Here, the lesion position images LI21, LI12 are illustrated in the first color, and the lesion position images LI11, LI22, LI13, LI23 are illustrated in the second color. This configuration makes it possible to easily specify the position of the lesion in the Z-direction.

The lesion position image LI is related to the information about the coordinate position, or the like, of the portion corresponding to the specific site of the heart (the position of the sinus node, the position of the atrioventricular node, the orientation of the His bundle, the position of the Purkinje fiber, etc.). As the lesion position image LI is generated by using the same first magnetic field information (biomagnetic field information) as that for the three-dimensional magnetic field strength distribution model DM, these pieces of position information included in the three-dimensional magnetic field strength distribution model DM may be used to relate it to these pieces of position information.

### <5. Device Position Image PI>

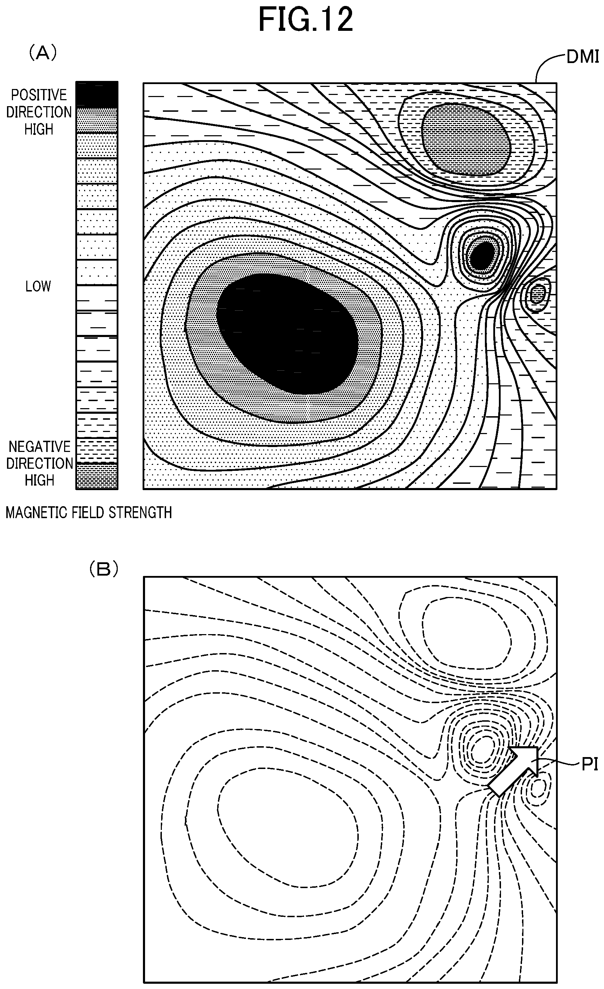

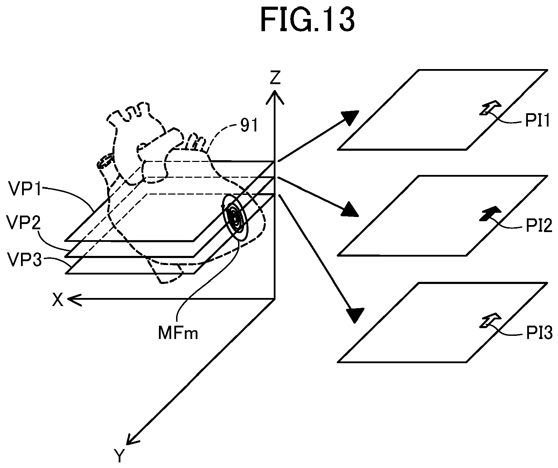

By using Fig. 11 to Fig. 13, the method for generating the device position image (catheter position image) PI by the device position image generation portion 524 will be described. Fig. 11 is an explanatory diagram schematically illustrating the method for detecting the living-body/device mixed magnetic field by the magnetic sensor array 10. Fig. 12(A) is an explanatory diagram illustrating a mixed magnetic field distribution image DMI. Fig. 12(B) is an explanatory diagram illustrating the device position image PI. Fig. 13 is an explanatory diagram illustrating the device position images PI on the plurality of virtual planes VP of the heart 91. Here, it is assumed that the main control portion 51 supplies the detection current to the marker 24 and the device magnetic field MFm occurs from the marker 24. The magnetic sensor array 10 outputs the second magnetic field information representing the strength and orientation of the living-body/device mixed magnetic field combining both the biomagnetic field MFh generated by the human body 90 and the device magnetic field MFm generated by the marker 24.

As the detection current flows through the marker 24, the device magnetic field MFm occurs from the marker 24, as illustrated in Fig. 11. Furthermore, the biomagnetic field (heart magnetic field) MFh occurs from the heart 91 due to the electric signal CD. The magnetic sensor array 10 detects the strength and orientation of the living-body/device mixed magnetic field combining the device magnetic field MFm and the biomagnetic field MFh. With the above-described configuration, the magnetic sensor array 10 may detect the strength and orientation of the living-body/device mixed magnetic field at each coordinate position in the three-dimensional space.

The device position information detection portion 514 compares the first magnetic field information with the second magnetic field information output from the magnetic sensor array 10 so as to specify the position of the marker 24. For example, the device position information detection portion 514 may generate the mixed magnetic field distribution image DMI illustrated in Fig. 12(A). The mixed magnetic field distribution image DMI in Fig. 12(A) represents, in a contour, the strength of the living-body/device mixed magnetic field at each position on the two-dimensional plane (XY plane). The contours in the mixed magnetic field distribution image DMI include a portion affected by the biomagnetic field MFh and a portion affected by the device magnetic field MFm. The device position information detection portion 514 compares the mixed magnetic field distribution image DMI with the magnetic field strength distribution image MI generated by the magnetic field strength distribution image generation portion 522 so as to specify the portion affected by the device magnetic field MFm. Specifically, the magnetic field strength distribution image MI is almost unaffected by the device magnetic field MFm and is formed by the biomagnetic field (heart magnetic field) MFh, while the mixed magnetic field distribution image DMI is formed by the biomagnetic field (heart magnetic field) MFh and the device magnetic field MFm. Therefore, based on the difference between these two images, it is possible to determine the portion affected by the device magnetic field MFm in the mixed magnetic field distribution image DMI. Thus, it is possible to specify the position of the marker 24 in the mixed magnetic field distribution image DMI. Therefore, based on the shape of the contour in the portion affected by the device magnetic field MFm, the orientation of the marker 24 at the specified position may be determined. Furthermore, by comparing the shapes of the contours in the portions affected by the device magnetic field MFm in the respective mixed magnetic field distribution images DMI on a plurality of virtual planes having different positions in the Z-direction, it is possible to determine the position, orientation, and inclination (rotation) of the marker 24 in three dimensions including the Z-direction as well as the position and orientation of the marker 24 on the XY plane.

After the device position information detection portion 514 specifies the position, orientation, and inclination (rotation) of the marker 24, the device position image generation portion 524 generates the device position image PI in Fig. 12(B). The device position image PI is an image in which an icon indicating the position of the distal end portion of the catheter 20 is located at the specified position of the marker 24. Here, an arrow shape is illustrated as the icon. The device position image PI uses the position and orientation of the arrow to indicate the position and orientation of the distal end portion of the catheter 20. Here, the device position image PI is represented as a stereoscopic image of the arrow, and by changing the shape of the arrow to an inclined shape, the inclination (rotation) of the distal end portion of the catheter 20 may be represented.

The device position image generation portion 524 may generate the device position image PI on the arbitrary virtual plane VP intersecting with the heart 91. Here, as an example, device position images PI1, PI2, PI3 corresponding to the three virtual planes (the first virtual plane VP1, the second virtual plane VP2, and the third virtual plane VP3), respectively, are illustrated. Here, the device position image generation portion 524 represents the position of the marker 24 in the Z-direction in the color of the device position image PI. Specifically, on the virtual plane (XY plane) VP that matches the position of the marker 24 in the Z-direction, the color of the inner side of the arrow in the device position image PI is illustrated in the first color, and on the virtual plane (XY plane) VP that does not match the position of the marker 24 in the Z-direction, the color of the inner side of the arrow in the device position image PI is illustrated in the second color. Here, the device position image PI2 is illustrated in the first color, and the device position images PI1, PI3 are illustrated in the second color. It can be understood that the position of the distal end of the catheter 20 in the Z-direction matches the second virtual plane VP2. This configuration makes it possible to easily specify the position of the distal end of the catheter in the Z-direction.

The device position image PI is related to the information about the coordinate position, and the like, of the portion corresponding to the specific site (the position of the sinus node, the position of the atrioventricular node, the orientation of the His bundle, the position of the Purkinje fiber, etc.) of the heart in the same manner as the lesion position image LI. As the device position image PI is generated by using the same first magnetic field information (biomagnetic field information) as that for the three-dimensional magnetic field strength distribution model DM, the pieces of position information included in the three-dimensional magnetic field strength distribution model DM may be used to relate it to these pieces of position information.

### <6. Synthetic Image CI>

By using Fig. 14, the method for generating the synthetic image CI by the synthetic image generation portion 52 will be described. Fig. 14 is a schematic diagram illustrating the method for generating the synthetic image CI. The synthetic image generation portion 52 superimposes, on the organ model image SI, one or more images out of the lesion position image LI, the magnetic field strength distribution image MI, and the device position image PI to generate the synthetic image CI in which one or more of the lesion position image LI, the magnetic field strength distribution image MI, and the device position image PI are superimposed and displayed on the organ model image SI. The lesion position image LI, the magnetic field strength distribution image MI, and the device position image PI each include the position information, and the like, on the specific site of the heart 91 (the position of the sinus node, the position of the atrioventricular node, the orientation of the His bundle, and the position of the Purkinje fiber). The synthetic image generation portion 52 may use these pieces of position information, and the like, to align the other three images with respect to the organ model image SI.

The generated synthetic image CI is displayed on the display screen 61. The synthetic image generation portion 52 continuously generates the synthetic images CI at a predetermined interval, and the display screen 61 displays the synthetic images CI in real time. Specifically, the display screen 61 displays, in real time, the biomagnetic field strength distribution, the relative position of the lesion, and the relative position of the distal end portion of the catheter 20 with respect to the three-dimensional heart model. The synthetic image generation portion 52 displays, on the display screen 61, the synthetic image CI corresponding to the arbitrary virtual plane VP in accordance with the operation of the operating portion 70.

### <7. Display Example of Display Screen>

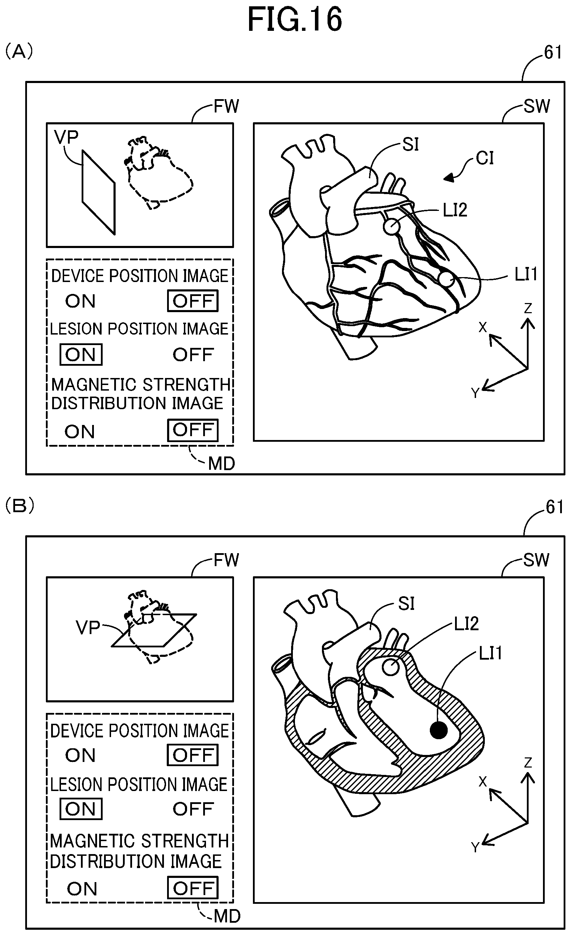

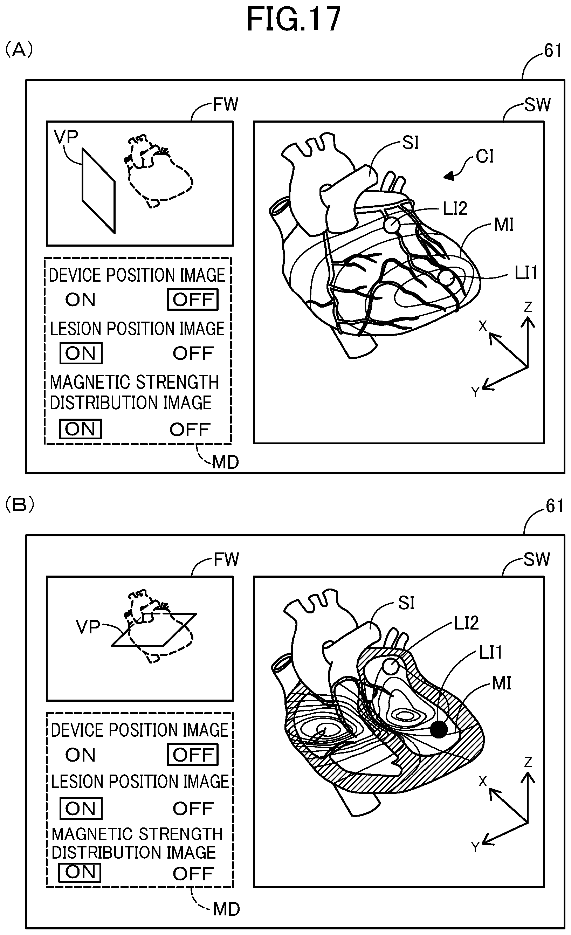

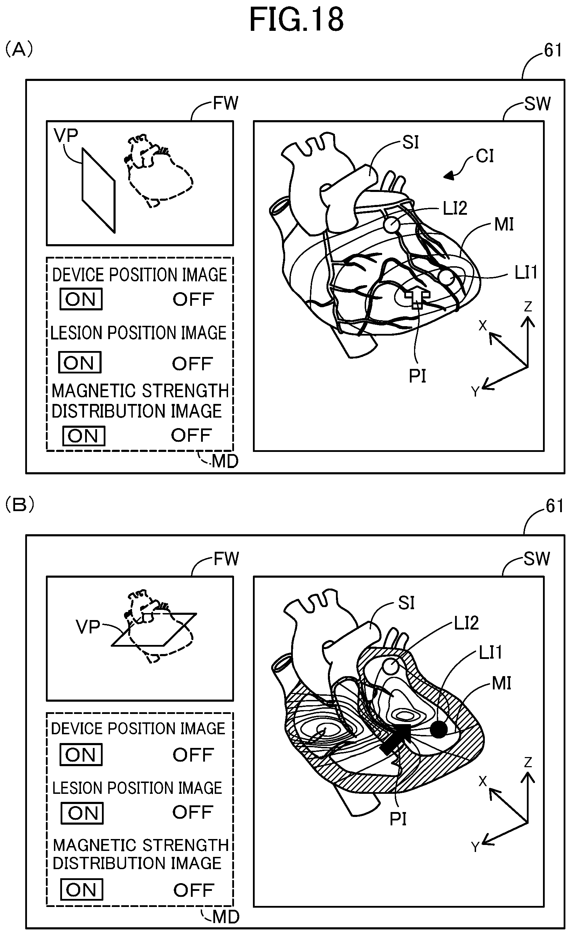

By using Fig. 15 to Fig. 18, the display example of the synthetic image CI on the display screen 61 will be described. Fig. 15 is an explanatory diagram illustrating the state of displaying the organ model image SI on the display screen 61. Fig. 15(A) and Fig. 15(B) have different display contents of the organ model image SI. Fig. 16 is an explanatory diagram illustrating the state of displaying the synthetic image CI of the organ model image SI and the lesion position image LI. Fig. 16(A) and Fig. 16(B) have different display contents of the synthetic image CI. Fig. 17 is an explanatory diagram illustrating the state of displaying the synthetic image CI of the organ model image SI, the lesion position image LI, and the magnetic field strength distribution image MI. Fig. 18 is an explanatory diagram illustrating the state of displaying the synthetic image CI of the organ model image SI, the lesion position image LI, the magnetic field strength distribution image MI, and the device position image PI.

As illustrated in Fig. 15(A) and Fig. 15(B), the display screen 61 displays a display menu MD, a first window FW, and a second window SW. The display menu MD has the display as to whether each of "device position image", "lesion position Image", and "magnetic field strength distribution image" is being displayed on the second window SW. In Fig. 15(A) and Fig. 15(B), the displays of "device position image", "lesion position image", and "magnetic field strength distribution image" are all "OFF". "ON" and "OFF" of "device position image", "lesion position image", and "magnetic field strength distribution image" may be switched (selected) by the operation of the operating portion 70.

The first window FW displays the heart image and the virtual plane VP so as to set the orientation and cross-sectional position of the heart represented by the organ model image SI displayed in the second window SW. In Fig. 15(A), the virtual plane VP does not intersect with the heart image in the first window FW, and the second window SW displays the synthetic image CI representing the outer surface of the heart viewed from the same position as the heart image viewed from the virtual plane VP. In Fig. 15(B), the virtual plane VP intersects with the heart image in the first window FW, and the second window SW displays the synthetic image CI representing the cross-section of the heart in the portion where the virtual plane VP intersects with the heart image. The position, orientation, and inclination of the virtual plane VP in the first window FW may be changed by the operation of the operating portion 70.

The second window SW displays the synthetic image CI having the content set on the display menu MD and the first window FW. In Fig. 15(A) and Fig. 15(B), the displays of "device position image", "lesion position image", and "magnetic field strength distribution image" are all "OFF" in the display menu MD, and therefore the organ model image SI is displayed and the device position image PI, the lesion position image LI, and the magnetic field strength distribution image MI are not displayed. Furthermore, a configuration may be such that the display menu MD is operated by the operating portion 70 to make a selection as to whether the organ model image SI is displayed in the second window SW.

In Fig. 16(A) and Fig. 16(B), in the display menu MD, the display of "lesion position Image" is "ON" and the displays of "device position image" and "magnetic field strength distribution image" are "OFF". The first window FW in Fig. 16(A) has the same settings as those of the first window FW in Fig. 15(A), and the first window FW in Fig. 16(B) has the same settings as those of the first window FW in Fig. 15(B). The second windows SW in Fig. 16(A) and Fig. 16(B) display the synthetic image CI of the organ model image SI and the lesion position image LI. Specifically, in Fig. 16(A), the circular lesion position image LI indicating the position of the lesion is displayed in a superimposed manner on the outer surface of the heart represented by the organ model image SI. Here, the two lesion position images LI1, LI2 are displayed. In Fig. 16(A), as neither of the two lesions intersects with the virtual plane in the first window FW, the two lesion position images LI1, LI2 are both in white. In Fig. 16(B), the circular lesion position image LI indicating the position of the lesion is displayed in a superimposed manner on the cross-section of the heart represented by the organ model image SI. In Fig. 16(B), as one of the two lesions intersects with the virtual plane in the first window FW, the lesion position image LI1 corresponding to the intersecting lesion is in black, and the lesion position image LI2 corresponding to the lesion that does not intersect is in white.

In Fig. 17, in the display menu MD, the displays of "lesion position image" and "magnetic field strength distribution image" are "ON" and the display of "device position image" is "OFF". Therefore, the second window SW displays the synthetic image CI of the organ model image SI, the lesion position image LI, and the magnetic field strength distribution image MI. In Fig. 17(A), the magnetic field strength distribution image MI representing the biomagnetic field strength in a contour is displayed in a superimposed manner on the outer surface of the heart represented by the organ model image SI. Furthermore, in Fig. 17(A), as in Fig. 16(A), the circular lesion position images LI1, LI2 are displayed at the positions where the lesions exist in the organ model image SI. By the operation of the operating portion 70, it is possible to set which one of the magnetic field strength distribution image MI and the lesion position images LI1, LI2 is displayed on the front side. In Fig. 17(B), the magnetic field strength distribution image MI representing the biomagnetic field strength in a contour is displayed in a superimposed manner on the cross-section of the heart represented by the organ model image SI. Furthermore, as in Fig. 16(B), the circular lesion position images LI1, LI2 are displayed at the positions where the lesions exist in the organ model image SI.

In Fig. 18, in the display menu MD, the displays of "lesion position Image", "magnetic field strength distribution image", and "device position image" are all "ON". Therefore, the second window SW displays the synthetic image CI of the organ model image SI, the lesion position image LI, the magnetic field strength distribution image MI, and the device position image PI. In Fig. 18(A), the arrow-shaped device position image PI indicating the position of the catheter 20 is displayed in a superimposed manner on the outer surface of the heart represented by the organ model image SI. In Fig. 18(A), as the distal end portion (the marker 24) of the catheter 20 does not intersect with the virtual plane in the first window FW, the device position image PI is in white. Furthermore, in Fig. 18(A), as in Fig. 17(A), the circular lesion position images LI1, LI2 are displayed at the positions where the lesions exist in the organ model image SI, and the magnetic field strength distribution image MI representing the biomagnetic field strength in a contour is displayed in a superimposed manner on the outer surface of the heart represented by the organ model image SI. In Fig. 18(B), the arrow-shaped device position image PI indicating the position of the catheter 20 is displayed in a superimposed manner on the cross-section of the heart represented by the organ model image SI. In Fig. 18(B), as the distal end portion (the marker 24) of the catheter 20 intersects with the virtual plane of the first window FW, the device position image PI is in black.

### <8. Effect Example of Present Embodiment>

In the medical apparatus 1 according to the present embodiment described above, as illustrated in Fig. 16 to Fig. 18, the display screen 61 displays the synthetic image CI including the three-dimensional or two-dimensional organ model image SI of the heart 91 and the lesion position image LI. Therefore, the technician of the catheter 20 may specify the position of the lesion and provide treatment while checking the displayed synthetic image CI. Furthermore, it is possible to check the presence or absence of the lesion after treatment without removing the catheter 20 after treatment. Thus, the medical apparatus 1 according to the present embodiment may improve the technique of displaying the state of the organ including the lesion.

Furthermore, in the medical apparatus 1 according to the present embodiment, as illustrated in Fig. 17 and Fig. 18, the synthetic image CI further includes the three-dimensional or two-dimensional magnetic field strength distribution image MI of the heart 91, and the display screen 61 displays the synthetic image CI including the magnetic field strength distribution image MI, the organ model image SI, and the lesion position image LI. Thus, it is possible to provide the technician of the catheter 20 with the biomagnetic field distribution of the heart 91 in real time.

Further, in the medical apparatus 1 according to the present embodiment, as illustrated in Fig. 18, the synthetic image CI further includes the device position image PI indicating the position of the catheter 20, and the display screen 61 displays the synthetic image CI including the device position image PI, the magnetic field strength distribution image MI, the organ model image SI, and the lesion position image LI. Thus, it is possible to provide the technician of the catheter 20 with the relative position of the catheter 20 with respect to the lesion (arrhythmia occurrence point) in real time.

Furthermore, with the medical apparatus 1 according to the present embodiment, as illustrated in Fig. 16 to Fig. 18, the display screen 61 may display the heart viewed at any angle by the synthetic image CI. Thus, the technician of the catheter 20 may specify the position of the lesion and provide treatment while checking the necessary portion.

### <9. Modification of Present Embodiment>

The disclosed embodiments are not limited to the embodiments described above. For example, the following modifications are possible.

### [Modification 1]

In the description according to the present embodiment, the biomagnetic field information acquisition portion 511 acquires the biomagnetic field information from the magnetic sensor array 10. However, the biomagnetic field information acquisition portion 511 may include a sensor that acquires the biomagnetic field from the human body 90. Furthermore, according to the present embodiment, the medical apparatus 1 includes the CT device 40. However, the medical apparatus 1 may omit the CT device 40. For example, the medical apparatus 1 may acquire the image information from a CT device outside the medical apparatus 1 or may acquire the image information from a storage medium storing the image information. Furthermore, the medical apparatus 1 may include an MRI device instead of the CT device 40. The image information acquisition portion 515 may acquire the image information including an MRI image, and the model image generation portion 521 may generate the organ model image SI and the three-dimensional heart model OM from the image information including the MRI image. An existing technique is applicable as the technique of generating the three-dimensional organ model OM from the image information including the MRI image. Furthermore, according to the present embodiment, the medical apparatus 1 includes the electrocardiograph 80. However, the medical apparatus 1 may omit the electrocardiograph 80. For example, the medical apparatus 1 may acquire the electrocardiographic information from an electrocardiograph outside the medical apparatus 1. Further, the medical apparatus 1 may not acquire the electrocardiographic information. Further, the medical apparatus 1 may omit the healthy subject biopotential magnetic information. Even in this case, the abnormality of the magnetic field is specified from the biomagnetic field information (the first magnetic field information) so as to detect the lesion position information.

### [Modification 2]

The synthetic image CI is displayed such that at least one of the lesion position image LI, the magnetic field strength distribution image MI, and the device position image PI is superimposed on the organ model image SI. However, in the synthetic image CI, each of the organ model image SI, the lesion position image LI, the magnetic field strength distribution image MI, and the device position image PI may be represented apart. Furthermore, on the display screen 61, the organ model image SI, the lesion position image LI, the magnetic field strength distribution image MI, and the device position image PI may be displayed in different display areas. In this case, the entire image displayed on the display screen 61 is the synthetic image CI.

### [Modification 3]

In the device position image PI and the lesion position image LI, the mode (color) of the image changes in accordance with the difference in the position of the corresponding virtual plane VP. However, in the device position image PI and the lesion position image LI, the mode of the image may be unchanged and fixed even when the position of the corresponding virtual plane VP changes. Furthermore, the shapes of the device position image PI and the lesion position image LI may be switched as appropriate in accordance with the operation of the operating portion 70. The device position image PI may have a shape representing the shape of the distal end portion of the catheter 20. The synthetic image CI according to the present embodiment includes the one device position image PI corresponding to the distal end portion of the one catheter 20. However, the synthetic image CI may include the plurality of device position images PI corresponding to the distal end portions of the plurality of catheters, respectively. Here, by the operation of the operating portion 70, among the device position images PI, only the device position image PI corresponding to the selected catheter may be displayed. That is, the catheter of which the device position image PI is displayed may be switched by the operation of the operating portion 70. Furthermore, the device position images PI may have different shapes from each other.

### [Modification 4]

The content of the magnetic field strength distribution image MI illustrated according to the present embodiment is an example, and the content of the magnetic field strength distribution image MI is not limited to the content according to the above-described embodiment. For example, in the magnetic field strength distribution image MI according to the above embodiment, the strength of the biomagnetic field MFh is represented in a contour, but the strength of the biomagnetic field MFh may be represented using the difference in the color, a numerical value, or a line graph. Furthermore, the orientation of the biomagnetic field MFh may be represented by a triangle, symbol, etc. Furthermore, the magnetic field strength distribution image MI may be replaced with the image representing the flow and density of the current generated by the living body. Even in this case, it can be said that the image representing the flow and density of the current generated by the living body is the image indicating the strength of the biomagnetic field.

### [Modification 5]

The display example of the display screen 61 according to the present embodiment is an example, and displays other than the display example described above may be used. Part of the above-described display example may be undisplayed, and other images may be added. For example, the first window FW may be undisplayed, or the blood pressure and the image of the operating portion may be displayed.

### [Modification 6]

In the description, the medical apparatus 1 according to the present embodiment is used for arrhythmia treatment. However, the medical apparatus 1 may be used for treatments other than arrhythmia treatment. Furthermore, the medical apparatus 1 may also be used for treatment of organs other than the heart. For example, the medical apparatus 1 may be used for treatment of the brain. In this case, the magnetic sensor array 10 may have a hat shape that is worn by the human body 90 to be treated.

### [Modification 7]

In the description, the catheter 20 according to the present embodiment is a cautery catheter using plasma. However, the cautery method by the catheter 20 may be not only generation of plasma but also flowing of high-frequency currents or irradiation of lasers. Furthermore, it may be used for not only cauterization but also injection of drugs by puncture or for other applications.

### [Modification 8]

In the catheter 20 according to the present embodiment, the marker 24 and the distal tip 22 are configured as separate members. However, the marker 24 and the distal tip 22 may not be separate members. For example, a cauterization high-frequency current and a position-detection current are alternately applied to the distal tip 22 so that the distal tip 22 may be provided with the function of a marker. Furthermore, although the medical apparatus 1 according to the present embodiment includes the catheter 20, it may include a medical tool such as a guide wire, endoscope, or dilator instead of the catheter 20. In this case, the synthetic image CI may be used to display the relative position of the distal end portion of the medical tool with respect to the biomagnetic field distribution.

### [Modification 9]

In the description, the catheter 20 according to the present embodiment is configured to use the magnetic field generated when the current flows through the coil as the marker 24. However, the use of a permanent magnet as the marker 24 may eliminate the need of the current flowing through the coil as the marker 24 to check the distal end position of the catheter. Furthermore, the magnetic field strength generated by the permanent magnet is constant, and therefore the magnetic field strength that is supposed to be generated by the living body may be determined by obtaining the difference between the magnetic field strength detected by the magnetic sensor array 10 and the magnetic field strength generated by the permanent magnet. However, in a case where the permanent magnet is used as the marker 24, when the magnetic field strength generated by the permanent magnet is extremely larger than the magnetic field strength generated by the living tissue, it is difficult to properly detect the magnetic field generated by the living tissue with the magnetic sensor array 10. Therefore, it is desirable that the magnetic field strength generated by the permanent magnet is equal to or less than 100 times the magnetic field strength generated by the living tissue.

### [Modification 10]

The configuration according to the present embodiment is also applicable to apparatuses other than medical apparatuses. For example, the configuration according to the present embodiment is also applicable to examination systems, examination methods, image generation apparatuses, image generation methods, etc.

Although the present mode has been described above based on the embodiment and the modifications, the embodiment of the mode described above is to facilitate understanding of the present mode and not to limit the present mode. The present mode may be modified or improved without departing from the scope of the claims. Furthermore, the technical feature thereof may be deleted as appropriate unless it is described as essential in the description.

### DESCRIPTION OF REFERENCE NUMERALS

1 Medical apparatus
10 Magnetic sensor array
20 Catheter
24 Marker
30 High-frequency generator
50 Computer
51 Main control portion
52 Synthetic image generation portion
60 Monitor
70 Operating portion
80 Electrocardiograph
90 Human body
91 Heart
511 Biomagnetic field information acquisition portion
512 Device magnetic field information acquisition portion
513 Lesion position information detection portion
514 Device position information detection portion
515 Image information acquisition portion
521 Model image generation portion
522 Magnetic field strength distribution image generation portion
523 Lesion position image generation portion
524 Device position image generation portion
MD Display menu
CI Synthetic image
MI Magnetic field strength distribution image
LI Lesion position image
PI Device position image
SI Organ model image
VP Virtual plane
FW First window
SW Second window

## Claims

1. A medical apparatus (1) comprising:
a biomagnetic field information acquisition portion (511) that is configured to acquire biomagnetic field information obtained from a biomagnetic field generated by an organ (91) including a lesion in a living body (90);
a lesion position information detection portion (513) that is configured to detect position information of the lesion in the organ from the acquired biomagnetic field information;
an image information acquisition portion (515) that is configured to acquire image information including an MRI image or CT image of the organ;
**characterized in that** it further comprises:
a device magnetic field information acquisition portion (512) that is configured to acquire device magnetic field information obtained from a magnetic field generated by a medical device (20) inserted into the living body (90);
a device position information detection portion (514) that is configured to detect position information of the medical device (20) in the living body (90) from the acquired device magnetic field information;
a synthetic image generation portion (52) that is configured to use the position information of the medical device, the image information, and the position information of the lesion to generate a synthetic image (CI) including an image (PI) representing the position of the medical device, a three-dimensional or two-dimensional organ model image (SI) of the organ, and an image (LI) representing the position of the lesion.

2. The medical apparatus (1) according to claim 1, wherein
the biomagnetic field information includes information about a magnetic field strength distribution of the biomagnetic field generated by the organ, and
the synthetic image generation portion (52) uses the biomagnetic field information, the image information, and the position information of the lesion to generate the synthetic image (CI) including a three-dimensional or two-dimensional magnetic field strength distribution image (MI) of the organ, the three-dimensional or two-dimensional organ model image (SI) of the organ, and the image (LI) representing the position of the lesion.

3. The medical apparatus (1) according to claim 1, wherein the biomagnetic field information is information output from a magnetic sensor (11) that is configured to detect the biomagnetic field generated by the organ (91).

4. The medical apparatus (1) according to claim 1 or 3, wherein
the device magnetic field information is information output from a magnetic sensor (11) that is configured to detect a magnetic field generated by a magnetic body (24) provided in a distal end of the medical device (20), and
the position of the medical device is a position of the magnetic body (24) of the medical device (20).

5. The medical apparatus (1) according to any one of claims 1 to 4, further comprising a display portion (60) that is conf igured to display the synthetic image generated by the synthetic image generation portion (52).

6. The medical apparatus (1) according to claim 5, further comprising an operating portion (70) that is conf igured to change a content of the synthetic image (CI) displayed on the display portion (60), wherein
when an operation is performed on the operating portion, the synthetic image generation portion (52) uses the image information and the position information on the lesion to generate a new synthetic image (CI) changed in accordance with the operation on the operating portion (70) and including the three-dimensional or two-dimensional organ model image (SI) of the organ and the image representing the position of the lesion (LI).

## Patentansprüche

1. Medizinische Vorrichtung (1) umfassend:
einen Biomagnetfeldinformationerfassungsabschnitt (511), der so eingerichtet ist, dass er Biomagnetfeldinformationen erfasst, die aus einem Biomagnetfeld erhalten sind, das von einem Organ (91) einschließlich einer Läsion in einem lebenden Körper (90) erzeugt ist;
einen Läsionspositionsinformationsdetektionssabschnitt (513), der so eingerichtet ist, dass er Positionsinformationen der Läsion in dem Organ aus den erfassten Biomagnetfeldinformationen detektiert;
einen Bildinformationserfassungsabschnitt (515), der so eingerichtet ist, dass er Bildinformationen einschließlich eines MRT-Bildes oder eines CT-Bildes des Organs erfasst;
**dadurch gekennzeichnet, dass** sie außerdem umfasst:
einen Gerätemagnetfeldinformationserfassungsabschnitt (512), der so eingerichtet ist, dass er Gerätemagnetfeldinformationen erfasst, die aus einem Magnetfeld erhalten sind, das von einem in den lebenden Körper (90) eingeführten medizinischen Gerät (20) erzeugt ist;
einen Gerätepositionsinformationsdetektionsabschnitt (514), der so eingerichtet ist, dass er Positionsinformationen des medizinischen Geräts (20) im lebenden Körper (90) aus den erfassten Gerätemagnetfeldinformationen detektiert;
einen Abschnitt (52) zum Erzeugen eines synthetischen Bildes, der so eingerichtet ist, dass er unter Verwendung der Positionsinformationen des medizinischen Geräts, der Bildinformationen und der Positionsinformationen der Läsion ein synthetisches Bild (CI) erzeugt, das ein Bild (PI), das die Position des medizinischen Geräts darstellt, ein dreidimensionales oder zweidimensionales Organmodellbild (SI) des Organs und ein Bild (LI), das die Position der Läsion darstellt, beinhaltet.

2. Medizinische Vorrichtung (1) gemäß Anspruch 1, wobei
die Biomagnetfeldinformationen Informationen über eine Magnetfeldstärkenverteilung des vom Organ erzeugten Biomagnetfeldes beinhalten und
der Abschnitt (52) zum Erzeugen eines synthetischen Bildes unter Verwendung der Biomagnetfeldinformation, der Bildinformationen und der Positionsinformationen der Läsion das synthetische Bild (CI) erzeugt, das ein dreidimensionales oder zweidimensionales Magnetfeldstärkeverteilungsbild (MI) des Organs, das dreidimensionale oder zweidimensionale Organmodellbild (SI) des Organs und das Bild (LI), das die Position der Läsion darstellt, beinhaltet.

3. Medizinische Vorrichtung (1) gemäß Anspruch 1, wobei die Biomagnetfeldinformationen Informationen sind, die von einem Magnetsensor (11) ausgegeben werden, der so eingerichtet ist, dass er das von dem Organ (91) erzeugte biomagnetische Feld detektiert.

4. Medizinische Vorrichtung (1) gemäß Anspruch 1 oder 3, wobei
die Gerätemagnetfeldinformationen Informationen sind, die von einem Magnetsensor (11) ausgegeben werden, der so eingerichtet ist, dass er ein Magnetfeld detektiert, das von einem Magnetkörper (24) erzeugt ist, der an einem distalen Ende des medizinischen Geräts (20) angeordnet ist, und
die Position des medizinischen Geräts eine Position des Magnetkörpers (24) des medizinischen Geräts (20) ist.

5. Medizinische Vorrichtung (1) gemäß einem der Ansprüche 1 bis 4, die außerdem einen Anzeigeabschnitt (60) umfasst, der so eingerichtet ist, dass er das vom Abschnitt (52) zum Erzeugen eines synthetischen Bildes erzeugte synthetische Bild anzeigt.

6. Medizinische Vorrichtung (1) gemäß Anspruch 5, die außerdem einen Bedienungsabschnitt (70) umfasst, der so eingerichtet ist, dass er einen Inhalt des auf dem Anzeigeabschnitt (60) angezeigten synthetischen Bildes (CI) ändert, wobei
wenn eine Bedienung an dem Bedienungsabschnitt durchgeführt wird, der Abschnitt (52) zum Erzeugen eines synthetischen Bildes unter Verwendung der Bildinformationen und der Positionsinformationen über die Läsion ein neues synthetisches Bild (CI) erzeugt, das gemäß der Bedienung an dem Bedienungsabschnitt (70) geändert ist und das dreidimensionale oder zweidimensionale Organmodellbild (SI) des Organs und das Bild, das die Position der Läsion (LI) darstellt, beinhaltet.

## Revendications

1. Appareil médical (1) comprenant :
une partie d'acquisition d'informations de champ biomagnétique (511) qui est configurée pour acquérir des informations de champ biomagnétique obtenues à partir d'un champ biomagnétique généré par un organe (91) incluant une lésion dans un corps vivant (90) ;
une partie de détection d'informations de position de lésion (513) qui est configurée pour détecter des informations de position de la lésion dans l'organe à partir des informations de champ biomagnétique acquises ;
une partie d'acquisition d'informations d'image (515) qui est configurée pour acquérir des informations d'image incluant une image IRM ou une image CT de l'organe ;
**caractérisé en ce qu'**il comprend en outre :
une partie d'acquisition d'informations de champ magnétique de dispositif (512) qui est configurée pour acquérir des informations de champ magnétique de dispositif obtenues à partir d'un champ magnétique généré par un dispositif médical (20) inséré dans le corps vivant (90) ;
une partie de détection d'informations de position de dispositif (514) qui est configurée pour détecter des informations de position du dispositif médical (20) dans le corps vivant (90) à partir des informations de champ magnétique de dispositif acquises ;
une partie de génération d'image synthétique (52) qui est configurée pour utiliser les informations de position du dispositif médical, les informations d'image et les informations de position de la lésion pour générer une image synthétique (CI) incluant une image (PI) représentant la position du dispositif médical, une image de modèle d'organe tridimensionnelle ou bidimensionnelle (SI) de l'organe et une image (LI) représentant la position de la lésion.

2. Appareil médical (1) selon la revendication 1, dans lequel
les informations sur le champ biomagnétique incluent des informations sur la distribution de l'intensité du champ magnétique du champ biomagnétique généré par l'organe, et
la partie de génération d'image synthétique (52) utilise les informations de champ biomagnétique, les informations d'image et les informations de position de la lésion pour générer l'image synthétique (CI) incluant une image de distribution d'intensité de champ magnétique tridimensionnelle ou bidimensionnelle (MI) de l'organe, l'image de modèle d'organe tridimensionnelle ou bidimensionnelle (SI) de l'organe et l'image (LI) représentant la position de la lésion.

3. Appareil médical (1) selon la revendication 1, dans lequel les informations de champ biomagnétique sont des informations délivrées par un capteur magnétique (11) qui est configuré pour détecter le champ biomagnétique généré par l'organe (91).

4. Appareil médical (1) selon la revendication 1 ou 3, dans lequel :
les informations de champ magnétique du dispositif sont des informations émises par un capteur magnétique (11) qui est configuré pour détecter un champ magnétique généré par un corps magnétique (24) prévu dans une extrémité distale du dispositif médical (20), et
la position du dispositif médical est une position du corps magnétique (24) du dispositif médical (20).

5. Appareil médical (1) selon l'une quelconque des revendications 1 à 4, comprenant en outre une partie d'affichage (60) qui est configurée pour afficher l'image synthétique générée par la partie de génération d'image synthétique (52).

6. Appareil médical (1) selon la revendication 5, comprenant en outre une partie de commande (70) qui est configurée pour modifier le contenu de l'image synthétique (CI) affichée sur la partie d'affichage (60), dans lequel
lorsqu'une opération est effectuée sur la partie opérationnelle, la partie de génération d'image synthétique (52) utilise les informations d'image et les informations de position sur la lésion pour générer une nouvelle image synthétique (CI) modifiée en fonction de l'opération sur la partie opérationnelle (70) et incluant l'image de modèle d'organe tridimensionnelle ou bidimensionnelle (SI) de l'organe et l'image représentant la position de la lésion (LI).
